# EUROPEAN PATENT APPLICATION

(11) **EP 1 923 704 A1**
(43) Date of publication of application: **21.05.2008**
(21) Application number: 06023954.8
(22) Date of filing: 17.11.2006
(51) Int. Cl.: G01N 33/68

(54) **Method of diagnosing vascular dementias and acute cerebral ischemia**

(71) Applicant: Friedrich-Alexander-Universität Erlangen-Nürnberg, 91054 Erlangen (DE); Georg-August-Universität Göttingen Stiftung des Öffentlichen Rechts (Bereich Humanmedizin), 37099 Göttingen (DE)
(72) Inventor: Bibl, Mirko, 37073 Göttingen (DE); Mollenhauer, Brit, 37073 Göttingen (DE); Esselmann, Hermann, 37308 Glasehausen (DE); Lewczuk, Piotr, 91341 Röttenbach (DE); Kornhuber, Johannes, 90491 Nürnberg (DE); Wiltfang, Jens, 91056 Erlangen (DE)
(74) Representative: Rehberg Hüppe + Partner

(57) **Abstract**

A method of diagnosing vascular dementias and acute stroke comprises the step of determining a concentration of at least one amyloid β peptide species in a blood sample taken from a patient. The at least one amyloid β peptide species is selected from the group consisting of Aβ1-37, Aβ1-38, Aβ1-39, Aβ1-42, and Aβ2-40. The concentration of the at least one amyloid β peptide species is compared to a threshold value for the respective one amyloid β peptide species; and a concentration of the least one amyloid β peptide species beyond the threshold value for the respective one amyloid β peptide species is taken as an indication of a vascular dementia or acute stroke in the patient from which the blood sample was taken.

## Description

### FIELD OF THE INVENTION

The invention relates to a method of diagnosing vascular dementias comprising the features of the preamble of independent claim 1. Particularly, the present invention relates to such a method which is suitable for discriminating vascular dementias from differential diagnostic relevant dementive disorders, such as Alzheimer's disease and depression. Further, the invention also relates to a similar method of diagnosing acute cerebral ischemia (stroke). Discriminating vascular dementias is a particular important point in course diagnosis of acute cerebral ischemia.

### ABBREVIATIONS USED

In this description, the following abbreviations are used: Aβ peptides = amyloid-beta peptides; Aβ-SDS-PAGE/immunoblot = amyloid-beta-sodium-dodecyl-sulphate-polyacrylamide-gel-electrophoresis with western immunoblot; AD = Alzheimer's disease; APP = beta-amyloid precursor protein; CCD-camera = charge coupled device camera; CSF = cerebrospinal fluid; DC = depressive control; DGN = Deutsche Gesellschaft für Neurologie; MMSE = Mini-Mental-Status Examination; NINCDS-ADRDA = National Institute Neurological and Communicative Disorders and Stroke-Alzheimer's Disease and Related Disorders Association; SDS = sodium dodecyl sulphate; %T = percentage of acrylamide; VAD = vascular dementias.

### DEFINITIONS

In this description, the use of the terms vascular dementia and Alzheimer's disease is based on the NINCDS-AIREN and NINCDS-ARDA criteria, respectively. The application of other criteria, like for example, the ADDTC criteria is also possible and leads to comparable results.

The term acute cerebral ischemia (stroke) is used according to the DGN guidelines which correspond to other standard definitions like for example in Victor and Adams: Principles of Neurology, current edition.

### PRIOR ART

The differential diagnosis among the two most common forms of dementia, Alzheimer's disease (AD) and vascular dementias (VAD), is crucial, since it does influence the therapeutic strategy. Thus far, multiparametric CSF-based neurochemical dementia diagnostics (CSF-NDD) may support the differential diagnosis of AD and VAD by measuring Aβ1-42, total tau and phospho-tau.

Van Oijen et al., 2006, reported that plasma Aβ peptides may have diagnostic value in incipient AD.

Gurol et al., 2006, reported that the plasma concentration of Aβ peptide species Aβ1-40 displayed relation to the load of vascular injuries in mild cognitive impairment, AD and cerebral amyloid angiopathy. Particularly, the plasma concentration of Aβ1-40 has been found to be associated with cerebral white matter lesions independently of potential confounders.

A quintet of Aβ peptide species, i.e. Aβ1-37/38/39/40/42, has been characterised as regular constituents of human CSF (Wiltfang et al., 2002; Lewczuk et al., 2004).

There are also some similarities in the clinic appearances of vascular dementias and of acute cerebral ischemia, including a high rate of disability, which interfere with the important early stage diagnosis of acute cerebral ischemia. Vascular dementias may appear during the course of acute cerebral ischemia.

### PROBLEM OF THE INVENTION

It is the problem of the present invention to provide an ex vivo method of diagnosing vascular dementias displaying good sensitivity and specificity so that it particularly enables discriminating vascular dementias from other kinds of dementia like, for example, Alzheimer's disease.

It is a further problem of the present invention to provide an ex vivo method of diagnosing acute cerebral ischemia suitable for early stage diagnosis.

### SOLUTION

The main problem of the invention is solved by a method comprising the features of independent claim 1. Preferred embodiments of this new method are defined in dependent claims 1 to 9.

The further problem of the invention is solved by a method comprising the features of independent claim 10. Preferred embodiments of this new method are defined in dependent claims 11 to 13.

### DESCRIPTION OF THE INVENTION

The inventors found the same quintet of Aβ peptide species, i.e. Aβ1-37/38/30/49/42, which have been characterised as regular constituents of human CSF before (Wiltfang et al., 2002; Lewczuk et al., 2004), in plasma. However, plasma Aβ peptides were characterised by the additional aminoterminally truncated species Aβ2-40.

Further and surprisingly, the inventors found that the plasma concentrations of the individual Aβ peptide species show characteristic variations in case of vascular dementias and acute cerebral ischemia.

Particularly in case of vascular dementias, in addition to an increase of Aβ1-40 the concentrations of Aβ1-37, Aβ1-38, Aβ1-39, Aβ1-42 and Aβ2-40 are decreased. The decrease is characteristic for vascular dementias in that it is not displayed with other differential diagnostic relevant dementive disorders, such as Alzheimer's disease and depression. This allows for comparing the plasma concentration of one or more of these last five Aβ peptide species with a threshold value for the respective Aβ peptide species. If the plasma concentration of the Aβ peptide species is below the threshold value for the respective Aβ peptide species, which is set below the typical plasma concentration of the respective Aβ peptide species without vascular dementias, this is an indication of a vascular dementia in the patient from which the plasma was taken.

The Aβ peptide species which display particularly high decreases in their plasma concentration with vascular dementias are Aβ1-38, Aβ1-42, and Aβ2-40, the most characteristic decrease being displayed by Aβ1-38.

With acute cerebral ischemia the plasma concentrations of the Aβ species display an inverted variation in that the concentrations of the Aβ species Aβ1-37, Aβ1-38, Aβ1-39, Aβ1-42 and Aβ2-40 are increased, whereas the concentration of the Aβ species Aβ1-40 is decreased. A strong dependency of this variation on the lapse of time between the stroke and the drawing of the blood sample is to be expected. The characteristic variations of the plasma concentrations of the individual Aβ species will only be very strong shortly after the actual stroke. This, however, is the most important time for diagnosing acute cerebral ischemia.

The absolute plasma concentrations of the Aβ peptide species may show a considerable variation which is not due to the occurrence of vascular dementias or acute cerebral ischemia. The relative plasma concentrations of the Aβ peptide species, however, display a much smaller variation without the occurrence of these diseases. Thus, it is preferred in the new method to compare a relative plasma concentration of the Aβ peptide species with a respective threshold value to more clearly discriminate vascular dementias from other kinds of dementia and to even more clearly diagnose acute cerebral ischemia.

For example, the relative plasma concentration of the Aβ peptide species can be determined as a fraction of the total Aβ peptide concentration or as a ratio of the absolute concentration of the Aβ peptide species and the absolute concentration of the Aβ peptide Aβ1-40.

If the fraction of the total Aβ peptide concentration of the Aβ peptide species Aβ1-38 is determined, a suitable threshold value for diagnosing vascular dementias is in a range of 7.6 % to 8.6 %. Preferably, this threshold value is in a range of 8.29 % to 8.43 %. A threshold value within the latter range is most suited to discriminate VAD from AD.

If the ratio of the absolute concentration of the Aβ peptide species Aβ1-38 and the absolute concentration of the Aβ peptide species Aβ1-40 is determined, a suitable threshold value for diagnosing vascular dementias is in a range of 0.116 to 0.156. Preferably, this threshold value is about 0.136.

A suitable threshold value for diagnosing acute cerebral ischemia based on the ratio of the absolute concentration of the Aβ peptide species Aβ1-38 and the absolute concentration of the Aβ peptide species Aβ1-40 is in a range of 0.136 to 0.24, preferably in a range of 0.185 to 0.22. The latter range does allow for discriminating acute cerebral ischemia from VAD, other dementias, AD and even from non-dementia patients.

As indicated before, the concentration of Aβ peptide species which will be determined in the new method will typically be a plasma concentration, i.e. the concentration will be determined in a blood plasma sample. As plasma is a component of blood, and as a blood plasma sample is a fraction of a blood sample taken from a patient, the concentrations of the Aβ peptide species determined in the new method may also be related to the total blood sample, or they may even be directly determined in the blood sample.

From the plasma or the total blood sample, the Aβ peptides are preferably enriched using an antibody specific to Aβ peptides, and then further analysed with regard to the concentration of the individual Aβ peptide species. The use of an n-terminal specific antibody for enriching the Aβ peptides proved as being particularly successful.

### SHORT DESCRIPTION OF THE DRAWINGS

In the following, the invention will be further described and explained by means of embodiment examples and with reference to the attached drawings.
- **Fig. 1**: is a graph showing the mean and the 95% confidence interval (Cl) of the ratio Aβ1-38/1-40 for three diagnostic groups consisting of DC, AD and VAD patients.
- **Fig. 2**: shows operator curves of the ratios Aβ1-38/1-40, Aβ1-42/1-40 and Aβ2-40/1-40, respectively, for the detection of VAD among all other patients.
- **Fig. 3**: is a graph similar to Fig. 1 but additionally showing the ratio Aβ1-38/1-40 for a patient with acute cerebral ischemia.

### DETAILED DESCRIPTION OF PREFERRED EMBODIMENTS OF THE INVENTION

13 patients with VAD (7 men, 6 women) were investigated in comparison to AD (n=10; 7 men, 3 women) and depressive controls (n=13; 6 men, 7 women. The clinical diagnosis was made according to DSM-IV and the respective current consensus criteria (Blacker et al., 1994; Roman et al., 1993). Moreover, VAD patients were characterized by typical signs of cerebrovascular disease in brain imaging (CT or MRI), whereas AD patients displayed mild white matter lesions at maximum. AD patients were further characterized by low CSF Aβ1-42 and high tau levels using the linear function (Aβ1-42=240+1.18xtau) from a large validation study of these biomarkers (Hulstaert et al., 1999). Patients with relevant cerebrovascular disease or clear focal atrophy in brain imaging, MMSE score below 26 or anamnesis of persistent cognitive decline for at least six month were excluded from the depressive control group.

Plasma samples were taken from all patients between 2000 and 2004. Handling of the samples followed a standardized protocol according to previously published data, except for time span until freezing which ranged up to 48 hours (Lewczuk et al., 2004). For quantification, Aβ peptides were enriched from plasma using the n-terminal specific immunoprecipitation by the monoclonal antibody 1 E8, and the Aβ-SDS-PAGE/immunoblot served for analysis as published elsewhere (Wiltfang et al., 2002; Bibl et al., 2004; Lewczuk et al., 2004). Samples were run as triplicates at minimum relative to dilution series of the synthetic Aβ1-37, Aβ1-38, Aβ1-39, Aβ1-40, Aβ1-42 and Aβ2-40 on each gel. The detection sensitivity of the Aβ-SDS-PAGE/immunoblot was 0.6-1 pg (Wiltfang et al., 2002; Bibl et al., 2004), Coefficients of variations of the Aβ peptides' concentrations ranged among 14.0 and 18.5% (Lewczuk et al., 2004). Quantifications of plasma Aβ peptides were obtained from individual blots and patient groups were characterized by mean and standard deviation. These data are listed in Table 1.

**Table 1:**

| Plasma Aβ peptide patterns, CSF Aβ1-42 and tau and demographic data of the diagnostic groups | | | | | | |
|---|---|---|---|---|---|---|
| **Diagnosis** | **DC (n=13)** | | **AD (n=10)** | | **VAD (n=13)** | |
| | MW | ±SD | MW | ±SD | MW | ±SD |
| **Age** | 62.62 | 7.68 | 69.60 | 8.86 | 71.00 | 6.73 |
| **MMSE** | 27.83 | 2.86 | 15.90 | 4.93 | 17.91 | 9.20 |
| **CSF Tau ELISA (ng/ml)** | 0.29 | 0.22 | 0.91 | 0.69 | 0.31 | 0.20 |
| **CSF Aβ1-42 ELISA (ng/ml)** | 0.94 | 0.46 | 0.33 | 0.11 | 0.48 | 0.21 |
| **Aβ1-37 (pg/ml)** | 25.30 | 7.33 | 23.60 | 5.40 | 21.10 | 7.07 |
| **Aβ1-38 (pg/ml)** | 33.79 | 13.18 | 32.57 | 12.18 | 30.53 | 11.95 |
| **Aβ1-39 (pg/ml)** | 28.04 | 7.57 | 29.58 | 8.24 | 27.29 | 9.83 |
| **Aβ1-40 (pg/ml)** | 223.6 | 88.3 | 219.4 | 111.3 | 272.2 | 118.1 |
| **Aβ1-42 (pg/ml)** | 26.49 | 7.10 | 24.41 | 4.82 | 18.65 | 8.29 |
| **Aβ2-40 (pg/ml)** | 20.09 | 5.62 | 21.20 | 3.70 | 15.22 | 5.70 |
| **total Aβ (pg/ml)¹** | 378.0 | 124.2 | 370.0 | 144.5 | 410.6 | 152.9 |
| **Aβ1-37%²** | 6.89 | 1.07 | 6.76 | 1.28 | 5.46 | 1.49 |
| **Aβ1-38% ²** | 8.84 | 1.12 | 8.82 | 0.38 | 7.43 | 0.85 |
| **Aβ1-39% ²** | 7.63 | 1.12 | 8.35 | 1.21 | 6.73 | 0.69 |
| **Aβ1-40% ²** | 57.64 | 6.54 | 56.89 | 7.36 | 64.74 | 6.12 |
| **Ap1-42% ²** | 7.24 | 0.99 | 7.17 | 1.89 | 4.65 | 1.69 |
| **Ap2-40% ²** | 5.52 | 1.15 | 6.32 | 1.92 | 3.82 | 1.02 |
| **Ratio Aβ1-38/1-40** | 0.155 | 0.027 | 0.158 | 0.022 | 0.116 | 0.019 |
| **Ratio Aβ1-42/1-40** | 0.128 | 0.030 | 0.132 | 0.051 | 0.074 | 0.032 |
| **Ratio Aβ2-40/1-40** | 0.099 | 0.031 | 0.117 | 0.052 | 0.060 | 0.020 |

| | | | | | | |
|---|---|---|---|---|---|---|
| ¹total Aβ peptide concentration as measured by the sum of all investigated Aβ peptides; ²percentage abundance of Aβ peptides relative to the sum of all investigated Aβ peptides. | | | | | | |

Plasma peptide values are indicated as absolute (pg/ml) or percentage (amount of each peptide relative to the sum of all single investigated Aβ peptides) and ratio (amount of each peptide relative to Aβ1-40) values, respectively. Significant group differences were evaluated in the Mann-Whitney U-test. The two-sided level of significance was taken as p<0.05. Receiver operating characteristic curve analysis was used to determine cut-off points at the maximum Youden index. The kappa coefficient was used for measurement of agreement between clinical diagnosis and neurochemical testing. Fisher's z-transformation served for comparison of kappa-values. The statistical software package SPSS, version 10.0 was used for computations.

The DC group was significantly younger than AD (p=4.9x10⁻²) and VAD (p=7x10⁻³). Age and MMSE score did not significantly differ between VAD (mean ± SD) and AD (mean ± SD). The average time until freezing did not differ among the diagnostic groups. Hypertension was prevalent in 23%, 30% and 38% of DC, AD and VAD, respectively. Hypercholesterinemia was prevalent in 31%, 20% and 15% of DC, AD and VAD, respectively.

None of the investigated Aβ peptides, neither in absolute, nor percentage terms, correlated with age, MMSE or the occurrence of hypertension. Prevalent hypercholesterolemia correlated weakly with higher percentage Aβ1-38 levels (p=4.3x10⁻²). The time until freezing strongly correlated with lower overall Aβ peptide levels (p<0.01), but most interestingly not with any percentage Aβ peptide amount. The female gender was weakly correlated with higher levels of Aβ1-39, Aβ1-40 and Aβ1-42 (p<0.05).

DC and AD displayed no significant differences in any of the investigated peptides, neither in absolute, nor percentage terms. In contrast, absolute Aβ1-42 (p=1.2x10⁻²) and Aβ2-40 (p=8x10⁻³) were decreased in VAD as compared to a combined group of DC and AD. In percentage terms, there was a decrease of Aβ1-37 (p=8x10⁻³), Aβ1-38 (p=6.9x10⁻⁵), Aβ1-39 (p=8.7x10⁻⁴), Aβ1-42 (p=8.2x10⁻⁵) and Aβ2-40 (p=6.9x10⁻⁵), paralleled by elevated amounts of Aβ1-40 (p=4.6x10⁻³) in VAD as compared to a combined group of DC and AD.

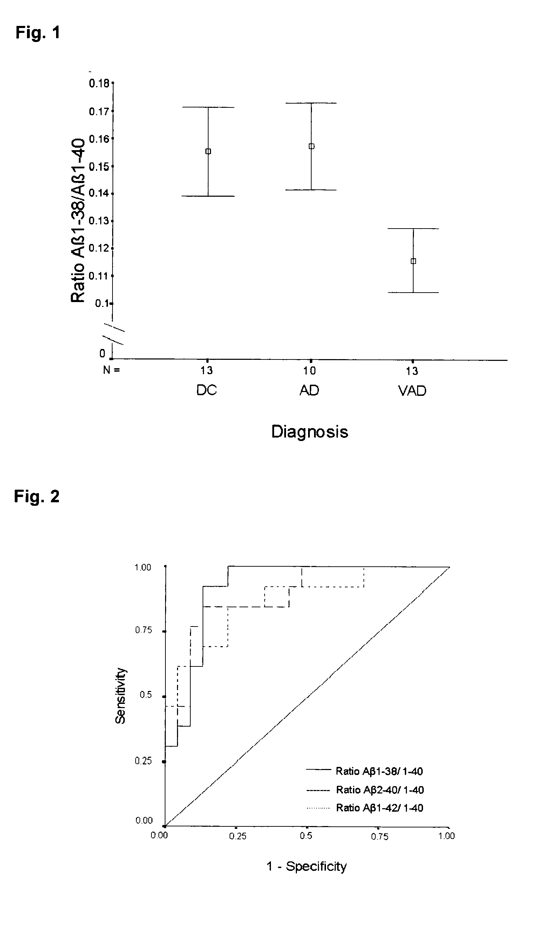

The most pronouncedly decreased peptides Aβ1-38, Aβ1-42 and Aβ2-40 were each combined with Aβ1-40 to the ratios Aβ1-38/ Aβ1-40, Aβ1-42/ Aβ1-40 and Aβ2-40/ Aβ1-40, respectively. **Fig. 1** visualizes the ratio Aβ1-38/ Aβ1-40 for all three diagnostic groups. ROC curve analysis as depicted in **Fig. 2** revealed areas under the curve (AUC) as follows: 0.92, 0.87 and 0.89 for Aβ1-38/ Aβ1-40, Aβ1-42/ Aβ1-40 and Aβ2-40/ Aβ1-40, respectively. Using a cut off point of 0.136, the ratio Aβ1-38/ Aβ1-40 exhibited a sensitivity of 92% for detection of VAD at a specificity of 87% in a combined group of AD and DC. The discriminative power of Aβ1-38/Aβ1-40 between VAD and AD was even higher, yielding a sensitivity and specificity of 92% and 90%, respectively, at the same cut off point.

When VAD was tested for absolute Aβ1-40 in comparison to a combined group of AD and DC, the kappa coefficient gave a value of 0.386. In contrast, kappa coefficient gave a value of 0.767 for the ratio Aβ1-38/Aβ1-40. After Ficher's z-transformation, the difference was significant to the level of 0.05.

In a further study, the Aβ peptide plasma concentrations were determined for a patient who had suffered an acute stroke within a maximum of 3 days. The stroke had been confirmed by MRT diagnosis. The Aβ peptide plasma concentrations which are listed in the following Table 2, however, clearly indicated the occurrence of an acute cerebral ischemia.

**Table 2:**

| Plasma Aβ peptide pattern for 1 Patient with acute cerebral ischemia (concentrations in ng/ml) | | | | | | | | | | | | | | | |
|---|---|---|---|---|---|---|---|---|---|---|---|---|---|---|---|
| **1-37** | **1-38** | **1-39** | **1-40** | **1-42** | **2-42** | **total Aβ** | **1-37 %** | **1-38 %** | **1-39 %** | **1-40 %** | **1-42 %** | **2-40 %** | **38/40** | **42/40** | **2-40/40** |
| 0,019 | 0,022 | 0,026 | 0,083 | 0,024 | 0,020 | 0,194 | 9,610 | 11,526 | 13,216 | 42,885 | 12,315 | 10,448 | 0,269 | 0,287 | 0,244 |

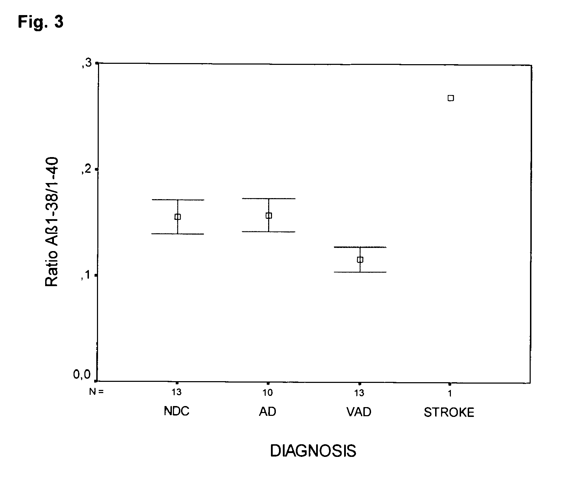

A comparison of the data listed in Tables 1 and 2, which is visualized in **Fig. 3** with regard to the ratio of Aβ1-38 and Aβ1-40, clearly indicates that the acute cerebral ischemia results in a variation of the regular Aβ peptide pattern (CD and AD groups) in an opposite direction as compared to VAD. Even considering an estimated SD of 10 %, the variation of the Aβ peptide pattern is so high that is allows for an easy diagnosis of acute cerebral ischemia based on the Aβ peptide pattern displayed in the plasma of a patient. A differentiation of a stroke with regard to VAD is particularly easy.

### DISCUSSION

The presented results on detailed characterization of Aβ peptide species in plasma of VAD in comparison to AD and depressive controls are consistent with previous studies (Irizarry, 2004) in respect of Aβ1-40 and Aβ1-42. Most interestingly, a recent publication by Gurol et al. (2006) reported a correlation of increased Aβ1-40 to the load of white matter lesions in MCI, AD and CAA. This effect turned out to be independent of potential confounders, such as age, sex, hypertension, diabetes mellitus, APO-E status, homocysteine, folate and B12. Increased Aβ1-40 was interpreted as a potential cerebrovascular risk factor, and combined with our results, it may be a potential biomarker to distinguish pure AD from mixed AD/VAD dementia or pure VAD, respectively. The specific role of Aβ1-40 in VAD is underlined by the fact that this peptide comprises the major component of vascular amyloid plaques, but is less abundant in senile AD plaques (Gravina et al., 1995). It remains an open question whether the reduction of plasma Aβ peptides other than Aβ1-40 may also be closely related to the cerebrovascular amyloid pathology. However, the diagnostic power of this effect can be increased, when Aβ1-40 is assessed in relation to other Aβ peptides in plasma.

### References

Bibl M, Esselmann H, Otto M, Lewczuk P, Cepek L, Rüther E, Kornhuber J. Cerebrospinal fluid (CSF) amyloid beta (Aβ) peptide patterns in Alzheimer's disease (AD) patients and non-demented controls depend on sample pre-treatment: Indication of carrier- mediated epitope masking of Aβ peptides. Electrophoresis 2004; 25:2912-2918.

Blacker D, Albert MS, Bassett SS, Go RC, Harrell LE, Folstein MF. Reliability an validity of NINCDS-ARDA criteria for Alzheimer's disease. The National Institute of Mental Health Genetics Initiative. Arch Neurol 1994; 51: 1198-204.

Gravina SA, Ho L, Eckman CB, Long KE, Otvos L, Younkin L, Suzuki N, Younkin SG. Amyloid β protein in Alzheimer's diesease brain. J Biol Chem 1995; 270: 7013-7016.

Gurol ME, Irizarry MC, Smith EE, Raju S, Diaz-Arrastia R, Bottiglieri T, Rosand J, Growdon JH, Greenberg SM. Plasma β-amyloid and white matter lesions in AD, MCI and cerebral amyloid angiopathy. Neurology 2006; 66:23-29.

Hulstaert F, Blennow K, lvanoiu A, Schoonderwald HC, Riemenschneider M, De Deyn PP, Bancher C, Cras P, Wiltfang J, Mehta PD, lqbal K, Pottel H, Vanmechelen E, Vanderstichele H. Improved discrimination of AD-patients using β-amyloid (1-42) and tau levels in CSF. Neurology 1999; 52:1555-1562.

Irizarry MC. Biomarkers of Alzheimer disease in plasma. NeuroRX 2004; 1:226-234.

Lewczuk P, Esselmann H, Bibl M, Paul S, Svitek J, Miertschischk J, Meyrer R, Smirnov A, Klein C, Bleich S, Sperling W, Kornhuber J, Rüther E, Wiltfang J. Electrophoretic separation of amyloid β (Aβ) peptides in plasma. Electrophoresis 2004; 25: 3336-3343.

Pomara N, Doraiswamy PM, Willoughby LM, Roth AE, Mulsant BH Sidtis JJ, Mehta PD Reynolds CF 3rd, Pollock BG. Elevation in plasma Abeta42 in geriatric depression: a pilot study. Neurochem Res 2006; 31:341-9

Roman GC, Tatemichi TK, Erkinjutti T, Cummings JL, Masdeu JC, Garcia JH, Amaducci L, Orgogozo JM, Brun A, Hofmann A. Vascular dementia: diagnostic criteria for research studies: report of the NINDS-AIREN International Workshop. Neurology 1993; 43:243-245.

Van Oijen M, Hofmann A, Soares HD, Koudstaal PJ, Breeteler MMB. Plasma Aβ1-40 and Aβ1-42 and the risk of dementia: a prospective case-cohort study. Lancet Neurol 2006; 5:655-660.

Wiltfang J, Esselmann H, Bibl M, Smirnov A, Otto M, Paul S, Schmid B, Klafki H-W, Mater M, Dyrks T, Bienert M, Beyermann M, Rüther E, Kornhuber J. Highly conserved and disease-specific patterns of carboxyterminally truncated Abeta peptides 1-37/38/39 in addition to 1-40/42 in Alzheimer's disease and patients with chronic neuroinflammation. J Neurochem 2002; 81: 481-496.

World Medical Organisation. Declaration of Helsinki. British Medical Journal 1996; 313:1448-1449.

## Claims

1. Method of diagnosing vascular dementias comprising the step of determining a concentration of at least one amyloid β peptide species in a blood sample taken from a patient, **characterized in that** the at least one amyloid β peptide species is selected from the group consisting of Aβ1-37, Aβ1-38, Aβ1-39, Aβ1-42, and Aβ2-40, that the concentration of the at least one amyloid β peptide species is compared to a threshold value for the respective one amyloid β peptide species, and that a concentration of the least one amyloid β peptide species below the threshold value for the respective one amyloid β peptide species is taken as an indication of a vascular dementia in the patient from which the blood sample was taken.

2. Method of claim 1, **characterized in that** the at least one amyloid β peptide species is selected from the group consisting of Aβ1-38, Aβ1-42, and Aβ2-40.

3. Method of claim 1 or 2, **characterized in that** the concentration of the at least one amyloid β peptide species is determined as a relative concentration.

4. Method of claim 3, **characterized in that** the relative concentration of the at least one amyloid β peptide species is determined as a fraction of the total amyloid β peptide concentration.

5. Method of claim 3, **characterized in that** the relative concentration of the at least one amyloid β peptide species is determined as a ratio of the absolute concentration of the at least one amyloid β peptide species and the absolute concentration of the amyloid β peptide species Aβ1-40.

6. Method of claim 4, **characterized in that** the at least one amyloid β peptide species is Aβ1-38, and that the threshold value is in a range of 7.6 % to 8.6 %, preferably in a range of 8.29 % to 8.43 %.

7. Method of claim 5, **characterized in that** the at least one amyloid β peptide species is Aβ1-38, and that the threshold value is in a range of 0.116 to 0.156, preferably in a range of 0.126 to 0.146.

8. Method of any of the claims 1 to 7, **characterized in that** the blood sample is a blood plasma sample.

9. Method of claim 10, **characterized in that** the amyloid β peptides are enriched from the blood plasma sample using an antibody to amyloid β peptides.

10. Method of diagnosing acute cerebral ischemia, particularly in a method of any of the claims 1 to 11, comprising the step of determining a concentration of at least one amyloid β peptide species in a blood sample taken from a patient, **characterized in that** the at least one amyloid β peptide species is selected from the group consisting of Aβ1-37, Aβ1-38, Aβ1-39, Aβ1-40, Aβ1-42, and Aβ2-40, that the concentration of the at least one amyloid β peptide species is compared to a threshold value for the respective one amyloid β peptide species, and that a concentration of the least one amyloid β peptide species selected from the group consisting of Aβ1-37, Aβ1-38, Aβ1-39, Aβ1-42, and Aβ2-40, which is above the threshold value for the respective one amyloid β peptide species, and/or a concentration of the amyloid β peptide species Aβ1-40, which is below the threshold value for the amyloid β peptide species Aβ1-40, is taken as an indication of an acute cerebral ischemia in the patient from which the blood sample was taken.

11. Method of claim 10, **characterized in that** the concentration of the at least one amyloid β peptide species is determined as a relative concentration.

12. Method of claim 11, **characterized in that** the relative concentration of the at least one amyloid β peptide species selected from the group consisting of Aβ1-37, Aβ1-38, Aβ1-39, Aβ1-42, and Aβ2-40 is determined as a ratio of the absolute concentration of the respective amyloid β peptide species and the absolute concentration of the amyloid β peptide species Aβ1-40.

13. Method of claim 12, **characterized in that** the at least one amyloid β peptide species is Aβ1-38, and that the threshold value is in a range of 0.136 to 0.24, preferably in a range of 0.185 to 0.22.
